# EUROPÄISCHE PATENTSCHRIFT

(11) **EP 1 067 129 B1**
(45) Veröffentlichungstag und Bekanntmachung des Hinweises auf die Patenterteilung: **27.08.2003**
(21) Anmeldenummer: 00113067.3
(22) Anmeldetag: 26.06.2000
(51) Int. Cl.: C07D 471/04, C07B 55/00

(54) **Racemisierung von R,S-Dioxo-benzylpyrrolopiperidin**
Racemisation of R,S-dioxo-benzylpyrrolopipieridine
La racemisation de la R,S-dioxo-benzylpyrrolopipéridine

(30) Priorität: 06.07.1999 DE 19931115
(43) Veröffentlichungstag der Anmeldung: 10.01.2001
(73) Patentinhaber: BAYER AG, 51368 Leverkusen (DE)
(72) Erfinder: Diehl, Herbert, Dr., 51373 Leverkusen (DE); Martin, Georg, Dr., 40764 Langenfeld (DE); Jaworek, Wilfried, Dr., 50996 Köln (DE); Krebs, Andreas, Dr., 51519 Odenthal (DE); Westen, Joachim, 42659 Solingen (DE)

(56) Entgegenhaltungen:
- EP-A- 0 550 903
- EP-A- 0 592 868
- GB-A- 1 569 486
- E. J. EBBERS ET. AL.: "Controlled Racemization of Optically Active Organic Compounds: Prospects for Asymmetric Transformation. " TETRAHEDRON, Bd. 53, Nr. 28, 1997, Seiten 9417-76, XP002149837 Oxford, GB

## Beschreibung

Die vorliegende Erfindung betrifft ein Verfahren zur Racemisierung von (1R,6S)-8-Benzyl-7,9-dioxo-2,8-diazabicyclo[4.3.0]nonan, hierin auch als R-DOPP oder R,S-Dioxo-benzylpyrrolopiperidin bezeichnet, durch Umsetzung mit einer Base.

Für die Herstellung des in der EP-A 350 733 beschriebenen Chinolonderivats wird enantiomerenreines (S,S)-2,8-Diazabicyclo[4.3.0]nonan, im folgenden auch als cis-S,S-Pyrrolopiperidin bezeichnet, benötigt, welches aus dem enantiomerenreinen (1S,6S)-8-Benzyl-2,8-diazabicyclo[4.3.0]nonan, im folgenden auch als S-BEPP oder S-Benzylpyrrolopiperidin bezeichnet, durch Debenzylierung erhalten werden kann. Es ist bekannt, S-BEPP über eine Racematspaltung mit Weinsäure aus racemischem cis-Benzylpyrrolopiperidin zu erhalten (EP-A 550 903). Das dabei anfallende Fehlenantiomer (R,R)-8-Benzyl-2,8-diazabicyclo[4.3.0]nonan, im folgenden auch als R-BEPP bezeichnet, kann nicht weiter verwendet werden. Nachteilig ist dabei der Verlust des an sich wertvollen Fehlenantiomers und der Aufwand für dessen Entsorgung.

Gemäß einem eigenen älteren Vorschlag (DE-A 199 27 412) kann die Racematspaltung auf einer früheren Stufe der Synthese des cis-S,S-Pyrrolopiperidins durchgeführt werden. Hierbei werden auf der Stufe des racemischen DOPP, im folgenden auch als rac-DOPP bezeichnet, die Enantiomeren durch Racematspaltung mit (-)-2,3:4,6-Di-O-isopropyliden-2-oxo-L-gulonsäure als deren Salz getrennt. Nach der Freisetzung aus den entsprechenden Salzen erhält man S-DOPP und R-DOPP.

Für die Racemisierung von R-DOPP und eine denkbare Rückführung des Fehlenantiomers sind mehrere Wege denkbar:

Zum einen könnten durch eine Dehydrierung des Fehlenantiomers zu einem Gemisch von Pyridin- oder Tetrahydropyridinderivat die beiden Stereozentren beseitigt werden und das Dehydriergemisch anschließend wieder zum Racemat hydriert werden. Es ist bereits bekannt, dass Pyridine aus den entsprechenden Piperidinen hergestellt werden können.

So beschreibt die GB-PS 1 157 001 ein Verfahren zur Herstellung von Pyridinen durch Umsetzung der entsprechenden Piperidine mit Sauerstoff und Ionen des Kupfers, Eisens oder Cobalts in der Flüssigphase bei 120 bis 150°C, wobei Essigsäure als Lösungsmittel verwendet wird.

Gemäß der EP-A 61 982 werden Pyridine und substituierte Pyridine durch Umsetzung der entsprechenden Piperidine in der Gasphase bei Temperaturen von 200 bis 500°C an einem Pd- oder Pt-Kontakt erhalten, wobei es sich bei den Substituenten um Alkyl- oder 1,5-Diaminopentan-Gruppen handeln kann.

Die US-A 4 051 140 beschreibt die Dehydrierung von Piperidinen in Gegenwart von Sauerstoff in der Gasphase an einem Vanadium-Kontakt bei 260 bis 540°C. Auch hierbei werden hauptsächlich Alkyl-substituierte Piperidine eingesetzt.

Die beschriebenen Gasphasen-Dehydrierungen benötigen hohe Reaktionstemperaturen, so dass eine Anwendung dieser Reaktion nur für bei diesen Temperaturen stabile Verbindungen in Frage kommt, was für R-DOPP nicht zutrifft. Das beschriebene Flüssigverfahren verlangt aufgrund des Einsatzes von Sauerstoff einen erhöhten Sicherheitsaufwand.

Bei allen Dehydrierungen wird vor der Möglichkeit einer Rückführung des Racemats in den Herstellungsprozess für das Chinolonderivat als zusätzlicher Reaktionsschritt die Hydrierung der erhaltenen Verbindung erforderlich.

Insgesamt kommt deshalb die Racemisierung durch Dehydrierung entweder fiir eine technische Anwendung im vorliegenden Fall grundsätzlich nicht in Frage oder sie ist wegen besonderem Sicherheitsaufwand und zusätzlichen Reaktionsstufen technisch aufwendig und nicht sinnvoll.

Zum anderen könnte die Racemisierung als eine basische Isomerisierung in Betracht gezogen werden. Die basische Racemisierung würde durch Abstraktion eines Protons an einem chiralen Zentrum und Ausbildung eines Carbanions erfolgen. Das Carbanion müßte durch eine oder mehrere benachbarte Keto-, Ester-, Nitril- und/oder Nitro-Gruppen stabilisiert werden. Eine nachfolgende Protonierung würde dann in der Regel das Racemat liefern. Solche Racemisierungen sind vor allem bei Aminosäuren und Aminosäurederivaten mit einem chiralen Zentrum bekannt (siehe Tetrahedron 53, 9417 (1997)).

Im Falle des R-DOPP müßten jedoch zwei chirale Zentren durch die Reaktion erfasst werden, weil im Falle einer nur partiellen Racemisierung mit der Bildung der entsprechenden trans-Verbindung gerechnet werden muss. Die basische Racemisierung kann deshalb nicht ohne weiteres auf die Racemisierung von R-DOPP übertragen werden.

Es wurde nun ein Verfahren zur Racemisierung von R-DOPP gefunden, das dadurch gekennzeichnet ist, dass man es mit einem Unterschuss an Base behandelt.

Zur erfindungsgemäßen Racemisierung kann man reines R-DOPP einsetzen oder Gemische, die überwiegend R-DOPP enthalten, beispielsweise Gemische, die über 75 Gew.-%, vorzugsweise über 80 Gew.-% R-DOPP enthalten. Ergänzend zu 100 Gew.-% können diese Gemische S-DOPP enthalten.

Als Basen für die erfindungsgemäße Racemisierung kommen beispielsweise Alkoholate in Frage, diese können der Formel (I) entsprechen

MOR (I),

in der
- M: für Lithium, Natrium oder Kalium und
- R: für geradkettiges oder verzweigtes C₁-C₆-Alkyl stehen.

In der Formel (I) steht M vorzugsweise für Natrium oder Kalium und R vorzugsweise für Methyl oder tert.-Butyl. Bevorzugte Einzelverbindungen der Formel (I) sind Natriummethylat, Natrium-tert.-butylat und Kalium-tert.-butylat. Besonders bevorzugt ist Kalium-tert.-butylat.

Die Alkoholate können in fester Form oder gelöst in einem Lösungsmittel zugeführt werden. Als Lösungsmittel kommen z.B. Alkohole und aprotische Lösungsmittel in Frage, beispielsweise der Alkohol, der dem jeweils eingesetzten Alkoholat entspricht und geradkettige, verzweigte und cyclische Ether sowie aromatische Kohlenwasserstoffe. Einzelbeispiele für aprotische Lösungsmittel sind: Methyl-tert.-butylether, Tetrahydrofuran, Dioxan, Toluol und Xylol. Bevorzugte Alkoholatlösungen sind: Kalium-tert.-butylat in tert.-Butanol und in Tetrahydrofuran und Natriummethylat in Methanol.

Wenn man Alkoholate in fester Form einsetzt oder eine geringe Menge einer konzentrierten Alkoholatlösung, so können Reaktionsgemische entstehen, die nicht oder nur schlecht rührbar sind. In solchcn Fällen ist es erforderlich, dem Reaktionsgemisch eine gute Rührbarkeit zu geben, indem man eines oder mehrere Lösungsmittel zufügt, beispielsweise Alkohole und/oder Ether der oben beschriebenen Art.

Zur Durchführung der erfindungsgemäßen Racemisierung ist es nicht erforderlich, dass das eingesetzte R-DOPP und die eingesetzte Base vollständig gelöst vorliegen.

Es sollte jedoch soviel Alkohol und/oder aprotisches Lösungsmittel vorhanden sein, dass sich ein gut rührbares Reaktionsgemisch ergibt.

Je nach Wahl des Lösungsmittels kann in relativ hoch konzentrierten Reaktionsgemischen gearbeitet werden. Setzt man beispielsweise Tetrahydrofuran als Lösungsmittel ein, so kann darin die Konzentration an R-DOPP bis zu ca. 50 Gew.-% betragen.

Die Base kann beispielsweise in einer Menge von 1 bis 20 Mol-%, bezogen auf das eingesetzte R-DOPP, eingesetzt werden. Vorzugsweise liegt diese Menge bei 2 bis 15 Mol-%, insbesondere bei 3 bis 10 Mol-%.

Zur Minimierung von unerwünschten Nebenreaktionen ist es vorteilhaft die erfindungsgemäße Racemisierung unter weitgehendem Ausschluss von Sauerstoff durchzuführen. Man kann hierzu z.B. das Reaktionsgefäß vor der Beschickung mit den Reaktanden mit einem inerten Gas spülen und die Racemisierung unter einer Inertgasatmosphäre durchführen. Geeignete Inertgase sind z.B. Stickstoff und Edelgase wie Argon.

Die erfindungsgemäße Racemisierung kann z.B. bei Temperaturen unter 40°C durchgeführt. Nach tiefen Temperaturen hin ist zu beachten, dass man keine Temperaturen wählt, bei denen das Reaktionsgemisch nicht mehr gut rührbar ist. Bei welcher Temperatur eine ausreichende Rührbarkeit nicht mehr gegeben ist hängt im wesentlichen von der Art und Menge der vorhandenen Lösungsmittel ab. Gegebenenfalls kann durch routinemäßige einfache Vorversuche ermittelt werden, bei welcher Temperatur ein gegebenes Reaktionsgemisch nicht mehr gut gerührt werden kann. Bevorzugt arbeitet man bei Temperaturen im Bereich -10 bis +30°C.

Die erfindungsgemäße Racemisierung ist im allgemeinen nach längstens 5 Stunden beendet. Unter geeigneten Reaktionsbedingungen (z.B. entsprechender Wahl der Base, des Lösungsmittels und der Temperatur) kann die erforderliche Reaktionszeit wesentlich kürzer sein und beispielsweise nur 15 Minuten oder noch weniger betragen.

Die Aufarbeitung des nach der Racemisierung vorliegenden Reaktionsgemisches kann so erfolgen, dass man zunächst die eingesetzte Base neutralisiert, z.B. durch Zusatz einer organischen Säure, z.B. einer C₁-C₆-Carbonsäure, einer Mineralsäure, z.B. Schwefelsäure oder Phosphorsäure, von Kohlensäure oder eines sauren Ionenaustauschers. Die Menge der Säure bzw. des sauren Ionenaustauschers kann z.B. 0,9 bis 1,1 Äquivalente pro Äquivalent eingesetzte Base betragen. Vorzugsweise liegt diese Menge bei 0,97 bis 1,03 Äquivalenten pro Äquivalent eingesetzte Base, insbesondere setzt man die Säure bzw. den sauren Ionenaustauscher in äquivalenter Menge ein, bezogen auf die eingesetzte Base. Danach kann man das Lösungsmittel entfernen, z.B. durch Abziehen, gegebenenfalls unter vermindertem Druck. Es hinterbleibt dann ein Gemisch, das im wesentlichen rac-DOPP und das bei der Neutralisation angefallene Salz enthält.

Dieses Gemisch kann man beispielsweise auf zweierlei Weise weiter verarbeiten, indem man
a) es mit einem geeigneten Lösungsmittel, z.B. einem Alkohol oder einem aromatischen Kohlenwasserstoff bei erhöhter Temperatur aufnimmt, die klare Lösung abkühlt, dabei gegebenenfalls nach Animpfen mit festem rac-DOPP, das erhaltene rac-DOPP ausfällt, es abfiltriert und schließlich wäscht und trocknet oder
b) es mit einem geeigneten Lösungsmittel, z.B. einem Keton löst, das zurückbleibende Salz abfiltriert und die Lösung des rac-DOPP weiterverwendet.

Bevorzugte Lösungsmittel für den Weg a) sind Isopropanol und Toluol, ein bevorzugtes Lösungsmittel für den Weg b) ist Methyl-ethyl-keton.

Die erfindungsgemäße Racemisierung hat den Vorteil, dass sie vollständig abläuft, die zu befürchtende Bildung von trans-Verbindungen praktisch nicht beobachtet wird und auf technisch einfache Weise sehr effektiv durchführbar ist. Das Racemat kann wieder in den Herstellungsprozess für das Chinolonderivat eingesetzt werden, was die Herstellung dieses Chinolonderivats wesentlich effizienter macht, weil das an sich wertvolle Fehlisomer wiederverwendet werden kann. Die erfindungsgemäße Arbeitsweise erfordert außerdem nur einfache Chemikalien und keine besonderen Vorsichtsmaßnahmen.

### Beispiele

### Beispiel 1

In einem 1000 ml 4-Hals-Kolben wurden unter Stickstoff 292 g Tetrahydrofuran vorgelegt und 300 g eines Gemisches enthaltend 91,4 Gew.-% R-DOPP und 8,6 Gew.-% S-DOPP eingetragen. Es wurde gerührt bis eine klare gelbe Lösung vorlag. Anschließend wurden bei Raumtemperatur 11,65 g einer 32 gew.-%igen Lösung von Kalium-tert.-butylat (2,8 Mol-% bezogen auf die Summe von R-DOPP und S-DOPP) in Tetrahydrofuran zugegeben. Man erhielt eine orange gefärbte Lösung, die 1 Stunde bei Raumtemperatur gerührt wurde. Anschließend wurden 4,15 g Essigsäure zugegeben wobei die Farbe der Lösung von Orange nach Gelb umschlug und Kaliumacetat ausfiel. Die vorliegende Suspension wurde am Rotationsverdampfer vom Tetrahydrofuran befreit und anschließend in 183 g Isopropanol aufgenommen. Die bei 40°C klare Lösung wurde auf 28°C abgekühlt und mit rac-DOPP angeimpft. Danach begann das in der Lösung vorliegende rac-DOPP auszufallen, wobei sich eine schwer rührbare Suspension bildete. Es wurde auf 0°C abgekühlt und der Niederschlag abgesaugt. Der Filterkuchen wurde mit 60 g Isopropanol bei 0°C gewaschen und im Vakuum getrocknet. So wurden 225,5 g (= 87,8 % der Theorie) rac-DOPP erhalten, bei dem das Verhältnis von R-DOPP zu S-DOPP 49,9 : 50,1 betrug.

### Beispiel 2

In einem 500 ml 4-Hals-Kolben wurden unter Stickstoff 262 g Toluol vorgelegt und 50 g eines Gemisches eingetragen, das 96 Gew.-% R-DOPP und 4 Gew.-% S-DOPP enthielt. Man rührte bis eine klare gelbe Lösung vorlag. Anschließend wurden bei Raumtemperatur 3,56 g einer 30 gew.-%igen Lösung von Natriummethylat in Methanol zugegeben (das entspricht 9,9 Mol-% Natriummethylat bezogen auf die Summe von R-DOPP und S-DOPP). Man erhielt eine orange gefärbte Lösung, die 1 Stunde bei Raumtemperatur nachgerührt und anschließend mit 1,18 g Essigsäure versetzt wurde, wobei die Farbe der Lösung nach Gelb umschlug. Das ausfallende Natriumacetat wurde abfiltriert. Das Filtrat wurde am Rotationsverdampfer vom Toluol befreit. Man erhielt 47,1 g eines öligen Produkts, das bei Raumtemperatur kristallisierte. Es handelte sich dabei um rac-DOPP, das R-DOPP und S-DOPP im Verhältnis 44:55 enthielt. Die Ausbeute entsprach 84,5 % der Theorie.

### Beispiel 3

In einem 250 ml 4-Hals-Kolben wurden unter Stickstoff 50 g Tetrahydrofuran vorgelegt und 50 g eines Gemisches eingetragen, das S-DOPP und R-DOPP im Verhältnis 5,4:94,6 enthielt. Es wurde gerührt bis eine klare gelbe Lösung vorlag. Anschließend wurden bei Raumtemperatur 1,03 g Kalium-tert.-butylat zugegeben (= 4,8 Mol-%, bezogen auf die Summe von S-DOPP und R-DOPP). Man erhielt eine orange gefärbte Lösung die 4 Stunden nachgerührt und anschließend mit 1,18 g Essigsäure versetzt wurde. Die Farbe der Lösung schlug nach Gelb um. Das Reaktionsgemisch wurde am Rotationsverdampfer vom Tetrahydrofuran befreit und in Methylethylketon aufgenommen. Man erhielt 113 g einer 32,8 gew.-%igen Lösung, was einer Ausbeute von 80 % der Theorie entspricht. Das Verhältnis von R-DOPP zu S-DOPP lag bei 50,6 : 49,4.

### Beispiel 4

In einem 250 ml 4-Hals-Kolben wurden unter Stickstoff 150 ml Toluol vorgelegt und 24,0 g eines Gemisches eingetragen, das S-DOPP und R-DOPP im Enantiomerenverhältnis 0,2:99,8 enthielt. Man rührte bis einer klare gelbe Lösung vorlag und gab dann bei Raumtemperatur 4,3 g festes Natriummethylat hinzu (24,5 Mol-%, bezogen auf R-DOPP). Man erhielt eine orange gefärbte Lösung der nach 2-stündigem Rühren bei Raumtemperatur 1,4 g Essigsäure zugegeben wurden. Dabei schlug die Farbe nach Gelb um. Das Reaktionsgemisch wurde am Rotationsverdampfer vom Toluol befreit. Man erhielt 21,5 g rac-DOPP (Ausbeute 86,5 % der Theorie). Das Enantiomerenverhältnis von R-DOPP zu S-DOPP betrug 49,8 : 50,2.

### Beispiel 5

In einem Reaktionsrohr, gefüllt mit Glasringen, wurden innerhalb von 1 Stunde simultan a) 244,3 g einer 20 gew.-%igen methanolischen Lösung eines Gemisches, das S-DOPP und R-DOPP im Enantiomerenverhältnis 8:92 enthielt und b) 36 g einer 30 gew.-%igen Lösung von Natriummethylat in Methanol zugetropft. Nach einer Verweilzeit von 1 Stunde wurde die Reaktionslösung durch Zudosierung von Essigsäure neutralisiert. Das Verhältnis von S-DOPP zu R-DOPP im Reaktionsgemisch betrug danach 57,7 : 42,5.

### Beispiel 6

Im einem inertisierten Rührgefäß wurden 476,4g einer 50,3 gew.-%igen Lösung von R-DOPP in Tetrahydrofuran vorgelegt. Die klare, gelbe Lösung wurde auf -5°C abgekühlt. Anschließend wurden 27,5 g einer 20 gew.-%igen Lösung von Kalium-tbutylat in Tetrahydrofuran zugegeben. Die Farbe der Lösung schlug nach rot um. Es wurde 30 Minuten gerührt. Anschließend wurden 3,0 g Essigsäure zugegeben, worauf die Farbe wieder nach gelb umschlug und sich eine Trübung von ausfallendem Kaliumacetat bildete. Man erhielt 504,5 g einer 44,6 gew.-%igen Lösung von rac-DOPP in Tetrahydrofuran. Die Ausbeute betrug 93,9 % der Theorie. Das Enantiomerenverhältnis von R-DOPP zu S-DOPP betrug 48,9 : 51,1.

## Patentansprüche

1. Verfahren zur Racemisierung von (1R,6S)-8-Benzyl-7,9-dioxo-2,8-diazabicyclo[4.3.0]nonan (= R-DOPP), **dadurch gekennzeichnet, dass** man es mit einem Unterschuss an Base behandelt.

2. Verfahren nach Anspruch 1, **dadurch gekennzeichnet, dass** man R-DOPP in Form von Gemischen einsetzt, die es zu über 75 Gew.-% enthalten.

3. Verfahren nach Ansprüchen 1 bis 2, **dadurch gekennzeichnet, dass** man als Base Alkoholate der Formel (I) einsetzt
MOR (I)
in der
M für Lithium, Natrium oder Kalium und
R für geradkettiges oder verzweigtes C₁-C₆-Alkyl stehen.

4. Verfahren nach Anspruch 3, **dadurch gekennzeichnet, dass** man das Alkoholat in fester Form zugibt.

5. Verfahren nach Anspruch 3, **dadurch gekennzeichnet, dass** man das Alkoholat gelöst in einem Lösungsmittel zugibt.

6. Verfahren nach Ansprüchen 1 bis 5, **dadurch gekennzeichnet, dass** soviel Lösungsmittel vorhanden ist, dass ein gut rührbares Reaktionsgemisch vorliegt.

7. Verfahren nach Ansprüchen 1 bis 6, **dadurch gekennzeichnet, dass** die Base in einer Menge von 1 bis 20 Mol-%, bezogen auf eingesetztes R-DOPP eingesetzt wird.

8. Verfahren nach Ansprüchen 1 bis 7, **dadurch gekennzeichnet, dass** man es unter weitgehendem Ausschluss von Sauerstoff und bei Temperaturen unter 40°C durchführt.

9. Verfahren nach Ansprüchen 1 bis 8, **dadurch gekennzeichnet, dass** man das nach der Racemisierung vorliegende Reaktionsgemisch aufarbeitet, indem man die eingesetzte Base neutralisiert, danach das Lösungsmittel entfernt und das hinterbleibende Gemisch mit einem Alkohol oder einem aromatischen Kohlenwasserstoff bei erhöhter Temperatur aufnimmt, die klare Lösung abkühlt und so das erhaltene racemische DOPP ausfällt, es abfiltriert und schließlich wäscht und trocknet.

10. Verfahren nach Ansprüchen 1 bis 9, **dadurch gekennzeichnet, dass** man zunächst entsprechend Anspruch 9 verfährt und das nach der Entfernung des Lösungsmittels hinterbleibende Gemisch in einem Keton löst, das zurückbleibende Salz abfiltriert und das hergestellte racemische DOPP als ketonische Lösung weiterverwendet.

## Claims

1. Process for racemizing (lR,6S)-8-benzyl-7,9-dioxo-2,8-diazabicyclo-[4.3.0]nonane (= R-DOPP), **characterized in that** the compound is treated with a substoichiometric amount of base.

2. Process according to Claim 1, **characterized in that** R-DOPP is employed in the form of mixtures comprising more than 75% by weight of R-DOPP.

3. Process according to Claims 1 to 2, **characterized in that** the base used is an alkoxide of the formula (I)
MOR (I)
in which
M represents lithium, sodium or potassium and
R represents straight-chain or branched C₁-C₆-alkyl.

4. Process according to Claim 3, **characterized in that** the alkoxide is added in solid form.

5. Process according to Claim 3, **characterized in that** the alkoxide is added dissolved in a solvent.

6. Process according to Claims 1 to 5, **characterized in that** the amount of solvent present is such that a readily stirrable reaction mixture is obtained.

7. Process according to Claims 1 to 6, **characterized in that** the base is employed in an amount of from 1 to 20 mol%, based on the R-DOPP used.

8. Process according to Claims 1 to 7, **characterized in that** it is carried out with substantial exclusion of oxygen and at temperatures below 40°C.

9. Process according to Claims 1 to 8, **characterized in that** the reaction mixture which is present after racemization is worked up by neutralizing the base employed, then removing the solvent and taking up the remaining mixture in an alcohol or an aromatic hydrocarbon at elevated temperature, cooling the clear solution and precipitating the resulting racemic DOPP, filtering off the DOPP and then washing and drying it.

10. Process according to Claims 1 to 9, **characterized in that** initially the procedure of Claim 9 is adopted, and the mixture which remains after removal of the solvent is dissolved in a ketone, the salt that remains is filtered off and the racemic DOPP which has been prepared is used as a ketonic solution.

## Revendications

1. Procédé de racémisation de (lR,6S)-8-benzyl-7,9-dioxo-2,8-diazabicyclo[4.3.0]nonane (=R-DOPP), **caractérisé en ce qu'**on traite ce composé avec une quantité déficitaire de base.

2. Procédé suivant la revendication 1, **caractérisé en ce qu'**on met en oeuvre le R-DOPP sous forme de mélanges qui le contiennent à raison de plus de 75% en poids.

3. Procédé suivant les revendications 1 à 2, **caractérisé en ce qu'**on met en oeuvre, comme base, des alcoolates de formule (I)
MOR (I)
dans laquelle
M représente le lithium, le sodium ou le potassium et R représente un groupe alkyle en C₁-C₆ à chaîne droite ou ramifiée.

4. Procédé suivant la revendication 3, **caractérisé en ce qu'**on ajoute l'alcoolate sous forme solide.

5. Procédé suivant la revendication 3, **caractérisé en ce qu'**on ajoute l'alcoolate en solution dans un solvant.

6. Procédé suivant les revendications 1 à 5, **caractérisé par** la présence de solvant en quantité telle que le mélange réactionnel se laisse bien agiter.

7. Procédé suivant les revendications 1 à 6, **caractérisé en ce que** la base est mise en oeuvre à raison de 1 à 20 mol% par rapport au R-DOPP mis en oeuvre.

8. Procédé suivant les revendications 1 à 7, **caractérisé en ce qu'**il est réalisé en excluant largement l'oxygène et à des températures inférieures à 40°C.

9. Procédé suivant les revendications 1 à 8, **caractérisé en ce qu'**on traite le mélange réactionnel après la racémisation en neutralisant la base mise en oeuvre, en enlevant ensuite le solvant et en reprenant le mélange résiduel avec un alcool ou un hydrocarbure aromatique à température élevée, en refroidissant la solution claire et en précipitant ainsi le DOPP racémique obtenu, en filtrant et finalement en lavant et séchant ce dernier.

10. Procédé suivant les revendications 1 à 9, **caractérisé en ce qu'**on procède d'abord selon la revendication 9 et dissout dans une cétone le mélange qui reste après enlèvement du solvant, on sépare par filtration le sel restant et on utilise ultérieurement le DOPP racémique obtenu sous forme de solution cétonique.
